# FASCICULE DE BREVET EUROPEEN

(11) **EP 4 139 273 B1**
(45) Date de publication et mention de la délivrance du brevet: **08.05.2024**
(21) Numéro de dépôt: 21731260.2
(22) Date de dépôt: 11.05.2021
(51) Int. Cl.: C07C 67/327, C07C 67/54, C07C 67/60, C07C 57/07, C07C 69/54

(54) **PROCEDE DE VALORISATION DE LOURDS D'ACIDE ACRYLIQUE ET D'ESTERS DUDIT ACIDE PAR CRAQUAGE THERMIQUE AVEC CONDENSATION PARTIELLE**
VERFAHREN ZUR RÜCKGEWINNUNG VON SCHWEREN NEBENPRODUKTEN AUS ACRYLSÄURE UND ESTERN DIESER SÄURE DURCH THERMISCHES KRACKEN MIT PARTIELLER KONDENSATION
METHOD FOR RECOVERING HEAVY BY-PRODUCTS FROM ACRYLIC ACID AND ESTERS OF SAID ACID BY THERMAL CRACKING WITH PARTIAL CONDENSATION

(30) Priorité: 19.04.2020 FR 2005057
(43) Date de publication de la demande: 01.03.2023
(73) Titulaire: ARKEMA FRANCE, 92700 Colombes (FR)
(72) Inventeur: FAUCONET, Michel, 57501 SAINT AVOLD cedex (FR); SANDRE, Frédéric, 57501 SAINT AVOLD cedex (FR)
(74) Mandataire: Arkema Patent
(86) Numéro de dépôt international: PCT/FR2021/050825
(87) Numéro de publication internationale: WO 2021/234249

(56) Documents cités:
- EP-A1- 0 717 031
- US-A1- 2004 225 149

## Description

### DOMAINE DE L'INVENTION

La présente invention concerne un procédé amélioré de régénération, par craquage thermique, d'un mélange de sous-produits lourds (résidus) issus d'une unité de production d'acide acrylique et d'une unité de production d'ester acrylique, conduisant à l'obtention d'acide acrylique (AA), d'esters acryliques (EA) et d'alcools, en vue de leur recyclage dans l'atelier de production de l'ester acrylique.

### ARRIERE-PLAN TECHNIQUE

Sous l'effet de la température durant les étapes de distillation, la fabrication d'acide acrylique et d'esters d'acide acrylique s'accompagne de la formation de composés lourds, dérivés d'addition de composés à propriété nucléophile sur la double liaison des monomères carbonylés insaturés, par réaction de Michael. On appelle composé "lourd" les composés dont le point d'ébullition est supérieur à celui du monomère acrylique fabriqué.

Le principal facteur limitant de l'efficacité de la régénération des composés dérivés de réaction de Michael contenus dans les flux lourds des ateliers d'AA et d'esters acryliques est l'augmentation de la viscosité du résidu lourd obtenu en pied de craqueur, lorsque la fraction riche en monomères acryliques et alcool générée par le craquage a été vaporisée.

La vaporisation de composés légers pendant le craquage entraîne une concentration des produits lourds dans le flux résidu, et une augmentation de la viscosité de ce flux. Cependant, le résidu doit rester suffisamment fluide après refroidissement pour être transporté puis traité en vue de sa destruction.

Dans le document EP 717 031, il a été montré qu'il est possible d'améliorer l'efficacité de la récupération de ces produits nobles valorisables, si le craquage est réalisé à partir d'un mélange de lourds provenant d'une unité de production d'AA et d'une unité de production d'ester acrylique, par rapport au craquage individuel des flux lourds de ces unités. L'effet de l'addition de lourds provenant des unités d'esters aux lourds provenant d'une unité AA est de diminuer la viscosité du résidu final. La réaction de craquage est réalisée à partir de mélanges à ratio de lourds AA / lourds ester de 9/1 à 1/9, à température de 180 à 220°C, sous pression atmosphérique, durant un temps de séjour de 0,5 à 3 heures. Dans ce procédé, le craquage et la vaporisation des composés légers générés sont réalisés dans un réacteur, puis le flux gazeux généré est envoyé dans une colonne à distiller et enfin le flux de pied de la colonne de distillation est recyclé dans le réacteur. En revanche, la fraction légère obtenue par craquage étant principalement constituée des monomères acryliques AA et ester, qui sont particulièrement sensibles à la polymérisation, l'étape de distillation doit nécessairement être opérée sous pression réduite, de façon à réduire la température, pour éviter la formation de polymère dans la colonne. Par ailleurs, les plateaux de rectification de la colonne à distiller provoquent la séparation efficace des inhibiteurs de polymérisation entraînés dans le mélange gazeux, qui refluent vers le fond de colonne, et par conséquent, il est nécessaire d'introduire des inhibiteurs de polymérisation frais en tête de colonne, pour éviter la formation de polymères en partie supérieure de la colonne. De ce fait, on doit séparer les étapes de réaction, réalisée sous pression plus élevée, et de distillation, réalisée sous pression réduite. L'installation pour réaliser le procédé doit donc être équipée d'un réacteur et d'un condenseur en tête, opérés à la même pression, et d'une colonne à distiller opérée à pression réduite, alimentée par le produit condensé, et comprenant un bouilleur en pied, et en tête un condenseur, un équipement de reflux et une alimentation d'inhibiteurs. Ce dispositif est compliqué et coûteux.

Du fait de la présence de forte concentration d'alcool dans les flux de synthèse des esters acryliques, une part importante des flux de lourds de ces unités de production est constituée des dérivés d'addition de l'alcool sur la double-liaison d'AA et de l'ester monomère. Par exemple, dans le cas de l'acrylate d'éthyle, il s'agit du 3-ethoxypropionate d'éthyle et de l'acide 3-éthoxypropionique. Ces composés alcoxy-propioniques comportent des liaisons C-O-C qui sont plus difficiles à casser que les liaisons C-C des autres dérivés. Dans les conditions de température habituellement utilisées pour la régénération de lourds acryliques, ils ne peuvent être dissociés qu'en présence de catalyseur en concentration importante. De ce fait, la quantité de produits valorisables à partir de lourds des unités d'esters acryliques est plus faible que celle d'unités d'acide acrylique. La quantité de composés potentiellement valorisables par craquage thermique est ainsi approximativement deux fois plus importante dans les lourds d'unité AA. Dans les plateformes de production de monomères acryliques, il existe par conséquent un intérêt de valoriser en priorité les lourds d'unité AA, par rapport aux lourds d'unités d'esters.

Toutefois, lorsque le ratio Lourds AA / Lourds EA (LAA/LEA) augmente, l'augmentation de la viscosité du résidu de la réaction de craquage est de plus en plus sensible aux paramètres permettant d'améliorer le rendement de craquage, à savoir une augmentation de la température de réaction et/ou une augmentation du temps de réaction. Cet effet est particulièrement important lorsque le ratio de lourds d'unité AA par rapport à la totalité du mélange de lourds AA et de lourds esters dépasse 50%.

En conséquence, il existe un besoin d'améliorer le rendement de régénération, par craquage thermique, des composés lourds issus des unités d'AA et d'esters contenant plus de 50% de lourds AA, en diminuant l'effet d'augmentation de viscosité du résidu pendant la vaporisation du mélange riche en monomère pendant le craquage.

Comme nous l'avons vu précédemment, l'efficacité de la régénération dépend essentiellement de deux paramètres : la température et le temps de séjour de la réaction. L'augmentation de ces deux paramètres tend à améliorer le rendement de régénération, mais cela se fait au détriment d'une augmentation de la viscosité du résidu de craquage.

Pour craquer efficacement des dérivés de Michael contenus dans les mélanges de LAA et LEA, une température minimale d'environ 140°C est nécessaire, mais à cette température, la durée de réaction doit être très longue si on veut obtenir un rendement acceptable. Dans le cas particulier de la dissociation thermique de dérivés de Michael à partir de mélanges de lourds AA et EA, une augmentation du temps de séjour à ce niveau de température relativement faible n'est pas favorable pour augmenter le rendement de récupération, parce qu'il existe une compétition entre la réaction de craquage des dérivés de Michael en monomères et celle de formation des dérivés de Michael à partir des monomères, cette dernière étant favorisée par les températures intermédiaires (140 - 160°C) et les temps de réaction importants. Il est donc préférable de réaliser le craquage à plus haute température (au-delà de 160°C, de préférence à plus de 180°C) et à plus faible temps de séjour (moins de 6h, de préférence moins de 4h).

Dans les plateformes de production d'AA et EA, il est très habituel d'avoir des fluctuations importantes de débits de production d'AA et d'EA, en fonction des demandes du marché, et corrélativement des fluctuations de débits de sous-produits lourds de ces unités. En conséquence, lorsque l'opération de craquage est réalisée par procédé en mode continu, on peut observer de fortes fluctuations du temps de séjour dans le réacteur de craquage, qui dépend du débit d'alimentation, et du ratio de lourds provenant des unités AA et esters.

Il existe par conséquent un intérêt de disposer d'un procédé de craquage de composés lourds des unités AA et EA permettant d'obtenir de bons rendements de valorisation, quelles que soient les fluctuations de débits et de ratios de lourds AA et lourds EA.

### RESUME DE L'INVENTION

L'invention concerne un procédé de régénération d'un mélange de sous-produits lourds provenant d'une unité de production d'acide acrylique et de sous-produits lourds provenant d'une unité de production d'ester acrylique de formule CH₂=CH-C(=O)-OR, R étant un groupement alkyle en C1-C8, ledit procédé comprenant les étapes suivantes :
i. introduire ledit mélange dans un réacteur et le soumettre à un craquage thermique produisant un flux de tête gazeux et un flux de pied (résidu) concentrés en produits lourds,
ii. soumettre ledit flux de tête à une condensation partielle conduisant à l'obtention d'un flux de tête contenant de l'acide acrylique, de l'ester acrylique et de l'alcool R-OH, destiné notamment au recyclage dans l'atelier de production d'ester acrylique, et d'un flux de pied,
iii. récupérer ledit résidu en vue d'un traitement d'élimination.

Avantageusement, les étapes i) et ii) sont réalisées à la même pression absolue, comprise entre 200 et 2000 hPa, de préférence entre 300 et 1500 hPa.

Selon diverses réalisations, ledit procédé comprend les caractères suivants, le cas échéant combinés.

Selon un mode de réalisation, l'opération de condensation partielle est réalisée dans un échangeur (condenseur) provoquant la condensation d'une partie de la vapeur générée lors du craquage thermique, sans que cette vapeur ne soit soumise préalablement à un fractionnement dans une colonne à distiller contenant un ou plusieurs plateaux de rectification.

Selon un mode de réalisation, la température du mélange gazeux (flux de tête) sortant de l'étape de condensation partielle est comprise entre 140°C et 180°C.

Selon un mode de réalisation, la température de craquage est comprise entre 140 et 260°C, de préférence entre 180 et 210°C.

Selon un mode de réalisation, le temps de séjour du mélange réactionnel dans le réacteur de craquage est compris entre 0,5h et 10h, de préférence entre 2h et 7h.

Selon un mode de réalisation, le flux de pied du réacteur (résidu) obtenu à l'issue de l'opération de craquage thermique présente une viscosité dynamique inférieure à 1 Pa.s, mesurée à température de 100°C par exemple à l'aide d'un viscosimètre Brookfield "CAP 1000+" de type cône - plan.

Selon un mode de réalisation, le flux de pied du condenseur partiel est au moins en partie recyclé dans le réacteur.

Selon un mode de réalisation, le flux liquide de pied du condenseur partiel est recyclé directement dans la phase liquide du réacteur, par tube plongeur dans le réacteur, ou dans une conduite de recirculation du milieu réactionnel à travers un échangeur externe, de préférence en amont de cet échangeur externe.

Selon un mode de réalisation, le flux de tête gazeux non condensé, issu du condenseur partiel, riche en AA, ester acrylique et alcool, est recyclé dans une unité de fabrication d'ester acrylique, de préférence sous forme liquide, après condensation dans un deuxième condenseur.

Selon un mode de réalisation, le ratio massique flux condensé / flux gazeux entrant dans le condenseur est compris entre 5% et 50%, de préférence entre 10% et 40%.

Selon un mode de réalisation, le ratio massique LAA/LEA est compris entre 3/7 et 9/1, de préférence entre 1/1 et 9/1.

Selon un mode de réalisation, ledit radical R est un groupement alkyle en C1-C4.

Selon un mode de réalisation, l'ester est de l'acrylate d'éthyle et l'alcool est l'éthanol.

Selon un mode de réalisation, ledit procédé de régénération est opéré en mode continu.

Selon un mode de réalisation, des inhibiteurs de polymérisation sont introduits au niveau du condenseur partiel et, lorsqu'il est employé, au niveau du deuxième condenseur. Ces inhibiteurs sont choisis parmi les inhibiteurs de polymérisation connus de l'homme de l'art : dérivés phénoliques comme l'hydroquinone et ses dérivés tels que l'éther méthylique de l'hydroquinone, le 2,6-di-terbutyl-4-méthyl phénol (BHT), et le 2,4-diméthyl-6-terbutyl phénol (Topanol A), la phénothiazine et ses dérivés, les sels de manganèse, comme l'acétate de manganèse, les sels de l'acide thiocarbamique ou dithiocarbamique, comme les thiocarbamates et dithiocarbamates métalliques, tels que le di-n-butyldithiocarbamate de cuivre, les composés N-oxyls, comme le 4-hydroxy-2,2,6,6- tétraméthylpipéridinoxyl (4-OH-TEMPO), les composés à groupements nitroso, tels que la N-nitroso phényl hydroxylamine et ses sels d'ammonium, les composés aminés comme les dérivés de paraphénylènediamine ou un mélange de ces inhibiteurs.

Selon un mode de réalisation, la réaction de craquage thermique a lieu en l'absence de catalyseur.

Selon un mode de réalisation, la réaction de craquage thermique a lieu en présence de catalyseurs. Comme catalyseurs, on peut citer des acides de Broensted, comme l'acide sulfurique, les acides sulfoniques, l'acide phosphorique, des zéolites, alumines ou catalyseurs à base de silice et alumine, des acides de Lewis comme par exemple des titanates, des bases, métaux alcalins ou leurs sels, des amines, phosphines, ou leur combinaison.

La présente invention permet de surmonter les inconvénients de l'état de la technique. Elle fournit plus particulièrement un procédé simplifié de craquage thermique d'un mélange de sous-produits lourds provenant d'une unité de production d'acide acrylique et de sous-produits lourds provenant d'une unité de production d'ester acrylique, permettant de récupérer efficacement des monomères valorisables dans la fabrication de l'ester acrylique. Cela est accompli grâce à l'association d'une étape de craquage thermique dudit mélange, et d'une étape de condensation partielle du flux de tête issu du craquage, les deux étapes étant effectuées à la même pression.

Avantageusement, le procédé de craquage de composés lourds des unités AA et EA selon l'invention permet d'obtenir de bons rendements de valorisation, quelles que soient les fluctuations de débits et de ratios de lourds AA et lourds EA.

Les avantages principaux du procédé selon l'invention sont :
- Une augmentation de rendement de récupération de produits nobles à partir des lourds dérivés de Michael présents dans les flux de lourds des ateliers AA et ester, en poussant plus à fond la réaction de craquage, sans limite à cause de la viscosité, et en traitant des mélanges de lourds AA et lourds esters plus riches en lourds AA ;
- Un procédé simple et peu coûteux en investissement puisqu'il ne nécessite pratiquement qu'un équipement supplémentaire (le condenseur partiel) simple à mettre en oeuvre (étape de condensation partielle à même pression que la réaction de craquage) ;
- Un procédé permettant de réduire les rejets en diminuant la quantité de résidu de craquage ;
- Un procédé permettant d'optimiser le rendement de récupération, malgré la fluctuation du temps de séjour et du ratio de lourds AA / lourds ester.

### FIGURES

La Figure 1 représente de manière schématique un mode de réalisation d'une installation selon l'invention.
La Figure 2 représente de manière schématique un autre mode de réalisation d'une installation selon l'invention.

### DESCRIPTION DE MODES DE REALISATION DE L'INVENTION

L'invention est maintenant décrite plus en détail et de façon non limitative dans la description qui suit.

Le terme « sous-produits lourds provenant d'une unité de production d'acide acrylique », comprend :
- des dérivés d'addition d'acide acrylique sur la double-liaison d'une autre molécule d'acide acrylique : acide 3-acryloxypropionique encore appelé "acide acrylique dimère" ou "dimère AA" ;
- des dérivés d'addition d'acide acrylique sur la double-liaison sur une molécule de dimère AA, pour former le "trimère AA" et autres oligomères formés par additions successives d'acide acrylique sur les doubles liaisons des oligomères AA précédents ;
- des dérivés d'addition d'acide carboxylique formés en sous-produits de l'acide acrylique (par exemple l'acide acétique) ou d'eau sur la double liaison de l'AA ou des oligomères cités précédemment.

Le terme « sous-produits lourds provenant d'une unité de production d'ester d'acide acrylique (EA)» comprend les mêmes composés que ceux cités précédemment, ainsi que :
- les esters d'alkyle de ces composés, le radical alkyle étant celui de l'ester EA;
- les dérivés d'addition de réaction de Michael résultant de l'addition d'alcool sur les double-liaison de l'acide acrylique, du dimère AA ou des oligomères AA, ainsi que de leurs esters.

Le terme "condensation partielle" fait référence à une opération au cours de laquelle le flux gazeux généré lors de l'étape de craquage thermique est partiellement condensé sous forme d'un flux liquide.

Le terme "condenseur partiel" fait référence à un équipement permettant de générer une fraction liquide (reflux) à partir d'un flux de vapeur chaud, par refroidissement sur une paroi plus froide que les vapeurs. Il s'agit en général d'un échangeur thermique maintenu plus froid que les vapeurs par la circulation d'un fluide réfrigérant. Le fluide réfrigérant peut être par exemple un flux gazeux, comme de l'air, de l'eau, ou tout autre flux liquide provenant d'un apport extérieur au procédé ou recyclé à partir d'un flux du procédé. La température des vapeurs en sortie de l'échangeur est régulée à la température souhaitée pour obtenir un flux plus ou moins important de liquide par condensation partielle, en modifiant la température et le débit du liquide réfrigérant en contact avec la paroi dans le condenseur partiel. Comme types d'échangeurs de chaleur on peut citer les échangeurs à tubes et calandre, constitués d'une coque et d'un faisceau de tubes à l'intérieur, dans lesquels les deux fluides (vapeurs générées dans le réacteur et fluide réfrigérant) circulent de manière séparée. On peut également citer les échangeurs spiralés ou les échangeurs à plaques, ou tout autre type d'échangeur connus de l'homme de l'art et permettant la condensation partielle de vapeurs. Ces échangeurs peuvent être des équipements horizontaux ou verticaux.

Les termes de "condensation totale" ou "condenseur total" font référence à une opération ou un équipement réalisant une condensation des composés condensables contenus dans les vapeurs non condensées en sortie du condenseur partiel, sous forme d'un liquide (distillat) qui est soutiré.

L'invention repose sur un procédé de craquage thermique, couplé à une opération de condensation partielle, les deux étapes étant réalisées à la même pression. Le procédé de l'invention s'applique à la régénération de monomères acryliques à partir d'un mélange de sous-produits lourds provenant d'une unité de production d'acide acrylique et de sous-produits lourds provenant d'une unité de production d'ester acrylique, et permet de récupérer efficacement ces monomères valorisables dans la fabrication de l'ester acrylique.

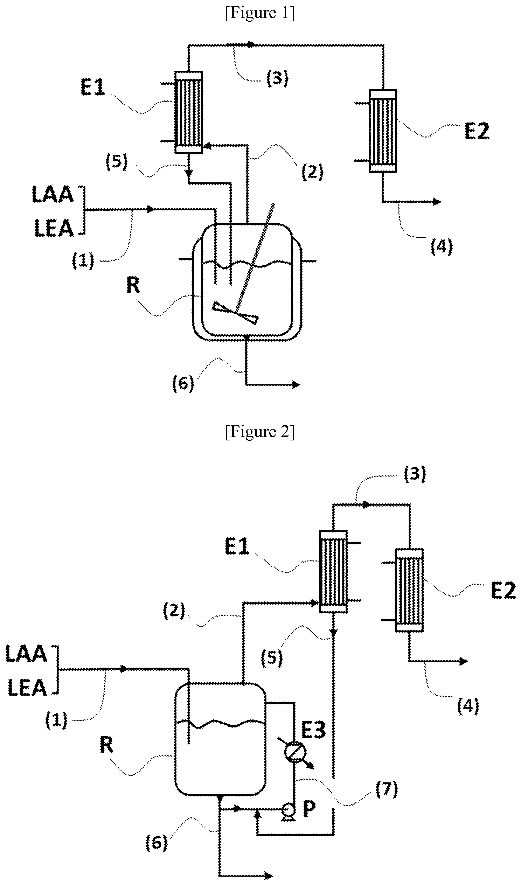

Selon le mode de réalisation du procédé représenté à la Figure 1, un mélange de flux provenant d'ateliers de production d'acide acrylique (LAA) et d'ester acrylique (LEA) est introduit en mode continu sous forme liquide dans le réacteur R. Ce mélange (1) est riche en composés lourds dérivés d'addition de Michael générés lors des étapes de synthèse et de purification des monomères correspondants (AA et EA), et contient également d'autres composés lourds accumulés lors des procédés de synthèse et purification, en particulier des inhibiteurs de polymérisation. Le mélange (1) est introduit dans le réacteur, où il est chauffé à la température requise pour réaliser le craquage des dérivés d'addition de Michael en composés plus légers qui sont extraits sous forme d'un mélange gazeux (2) en tête de réacteur. Ce flux gazeux est envoyé dans un condenseur partiel E1 constitué par exemple d'un échangeur à tubes et calandre. Ledit échangeur est circulé par de l'eau à une température et avec un débit adaptés pour refroidir la vapeur à la température requise, mesurée dans le flux (3) de vapeur non condensée en sortie de l'échangeur, et pour obtenir le ratio de condensation souhaité. Ce flux de vapeur non condensée riche en composés valorisables (AA, ester, alcool) et contenant quelques composés lourds en concentration faible, est avantageusement recyclé dans le procédé de production de l'ester EA, soit directement sous forme vapeur, ou après condensation totale dans un échangeur E2. Dans ce dernier cas, c'est un flux liquide (4) qui est recyclé dans l'unité de production d'ester EA.

En fond du condenseur partiel, on récupère un flux liquide (5) (appelé encore reflux) plus riche en composés lourds, en particulier des dérivés de Michael 3-alcoxy-propionique dont la température d'ébullition est intermédiaire entres celle des monomères et celles des composés les plus lourds, et contenant également un peu d'inhibiteurs de polymérisation. Ce flux (5) et renvoyé au moins en partie dans le réacteur, en l'introduisant de préférence directement dans la phase liquide du réacteur, via une conduite plongeante séparée ou via la conduite plongeante d'alimentation du réacteur en composés LAA et LEA.

Selon un mode non décrit sur la Figure 1, une partie du flux liquide condensé dans le condenseur partiel, contenant des inhibiteurs de polymérisation à faible concentration, est renvoyée à l'aide d'une pompe vers la sortie haute du condenseur partiel, de façon à protéger l'équipement contre un risque de polymérisation à l'intérieur du condenseur.

Le flux de résidu récupéré en fond de réacteur (6) est refroidi, puis éliminé sous forme d'un liquide de viscosité modérée, de façon à pouvoir être transporté sans difficulté par pompe, par exemple jusqu'à un stockage ou une unité d'incinération.

Selon un mode de réalisation non décrit sur la Figure 1, le flux d'alimentation composé de LAA et LEA est préchauffé à travers un échangeur de chaleur avant d'être introduit dans le réacteur.

Selon un autre mode de réalisation, la réaction de craquage thermique est réalisée en mode discontinu.

Selon un mode de réalisation, le flux d'alimentation est introduit directement dans la phase liquide du réacteur, via une conduite plongeante.

Le schéma de la Figure 2 décrit un mode de réalisation du procédé selon le même principe que celui de la Figure 1, en précisant une mise en oeuvre possible dans le cas où le réacteur est chauffé par recirculation, à l'aide d'une pompe P, du milieu réactionnel contenu dans le réacteur (7), à travers un échangeur de chaleur E3. Dans ce mode de réalisation, la fraction de flux liquide (5) renvoyée dans le réacteur sera avantageusement recyclée dans la boucle de recirculation, de façon préférentielle en amont de l'échangeur E3, par exemple en amont de la pompe de recirculation P.

### EXEMPLES

Les exemples suivants illustrent l'invention sans la limiter.

En référence aux indications de la Figure 1, dans ces exemples, la température de réaction est la température mesurée dans le liquide contenu dans la phase liquide du réacteur R, la "température de tête" est la température des vapeurs mesurée en sortie du condenseur partiel E1 (flux 3), le "temps de séjour" dans le réacteur est mesuré par le ratio massique du volume utile du réacteur (volume de phase liquide dans le réacteur) sur le débit d'alimentation des lourds (flux 1). Le "ratio d'étêtage" est le rapport massique du débit de flux condensé en sortie de condenseur total E2 (flux 4) sur le débit d'alimentation (flux 1), le "taux de récupération utile" est le rapport massique de la somme des composés valorisables (AA, AE et éthanol) recueillis dans le distillat après condensation totale (flux 4) sur le débit d'alimentation de lourds (flux 1). Le "taux de conversion utile" est le ratio massique de la somme des composés valorisables (AA, AE et éthanol) générés par craquage et récupérés dans le flux condensé totalement (flux 4), après soustraction de la somme de ces composés présents dans l'alimentation (flux 1), rapportée au débit d'alimentation du réacteur (flux1) après soustraction des composés valorisables présents dans ce flux. Enfin, la "viscosité @100°C" est la valeur de viscosité en Pa.s à température de 100°C du résidu obtenu en pied de réacteur (flux 6), mesurée à l'aide d'un viscosimètre Brookfield "CAP 1000+" de type cône - plan, équipé du mobile n°2, à vitesse de rotation de 750 rotations par minute.

Tous les essais ont été réalisés sous pression atmosphérique.

### Exemple 1 : Craquage avec condensation partielle et reflux du liquide condensé dans le ciel gazeux du réacteur

Le montage est constitué d'un réacteur en verre de volume total de 1 litre, chauffé par recirculation d'huile à travers sa double paroi, équipé d'une sortie latérale pour la sortie du résidu, assurant un volume utile de liquide contenu dans le réacteur de 240ml.

Le réacteur est équipé d'un agitateur, d'une sonde de température immergée dans la phase liquide, d'une conduite verticale en partie supérieure, pour l'extraction des vapeurs, et d'un condenseur partiel. La condensation partielle est assurée par refroidissement des vapeurs de réaction sur un doigt de gant placé à l'intérieur du tube vertical d'extraction des vapeurs, dans lequel circule de l'huile à température régulée de 150°C. Une sonde de température permet de mesurer la température des vapeurs non condensées en sortie du condenseur partiel. Ces vapeurs non condensées sont dirigées vers un condenseur total, constitué par un réfrigérant à double paroi circulée par de l'eau à 10°C. Le liquide (distillat) est recueilli dans un flacon récepteur et analysé. Les éléments de montage en contact avec les vapeurs, depuis leur génération dans le réacteur jusqu'à leur entrée dans le deuxième condenseur, sont calorifugés pour réduire les échanges thermiques avec l'extérieur. Dans ce montage, la fraction de vapeur condensée dans le condenseur partiel reflue naturellement vers le réacteur.

Un mélange constitué de 60% massique de composés lourds provenant d'une unité de production d'AA et 40% massique de composés lourds provenant d'une unité de production d'acrylate d'éthyle (AE) est envoyé en continu dans le réacteur, avec un débit de 70g/h. Dans ces conditions, le temps de séjour est de 3,4h. Le mélange d'alimentation est composé essentiellement de composés valorisables (9% AA et moins de 0,1% d'AE et d'éthanol), de composés lourds dérivés de Michael (35,5% de dimère AA (AA2), 1,5% de trimère AA (AA3), 3,3% d'acide 3-éthoxypropionique (AEP), 0,3% d'acide 3-hydroxypropionique (AHP), 6,2% de 3-éthoxypropionate d'éthyle (EPE), 6,7% de 3-acryloxypropionate d'éthyle (APE), 0,5% de 3-hydroxypropionate d'éthyle (HPE), et des inhibiteurs de polymérisation (2,7% d'hydroquinone (HQ), 0,6% de phénothiazine (PTZ)).

Dans l'exemple 1, après un fonctionnement en conditions stables pendant plus de 24h, la température de réaction est de 195°C et la température des vapeurs en sortie du condenseur partiel est de 157°C.

Le Tableau 1 résume les conditions opératoires et performances de ces essais.

### Exemple 2 et 3 (comparatifs) : (SF57-4 et SF58-4) Craquage sans condensation, selon l'art antérieur

Ces 2 exemples expérimentaux ont été réalisés dans le même montage que l'exemple 1 et dans des conditions identiques, hormis le fait que le doigt de gant interne est vide, ce qui supprime l'effet de refroidissement par circulation du fluide caloporteur.

Après un fonctionnement en conditions stables pendant plus de 24h, les résultats obtenus sont décrits dans le Tableau 1 ci-dessous.

**[Tableau 1]**

| | **selon l'invention** | **comparatif** | |
|---|---|---|---|
| N° essai | **1** | **2** | **3** |
| Temps de séjour | 3,5 h | 3,5 h | 6 h |
| Température Réaction | 195°C | 194°C | 187°C |
| Température tête | 157°C | 177°C | 168°C |
| Ratio d'étêtage | 58,4% | 61,1% | 57,8% |
| Taux de Récupération Utile | 43,2% | 41,7% | 37,3% |
| Taux de Conversion Utile | 37,6% | 35,9% | 31,0% |
| Viscosité du résidu en Pa.s @ 100°C | 0,318 | 0,451 | 0,380 |

Ces exemples comparatifs montrent l'intérêt d'une diminution de la température de la vapeur en sortie de réaction entrainant la condensation partielle de cette vapeur et un reflux liquide dans le réacteur, la conséquence étant une amélioration du rendement de récupération de composés nobles, avec une viscosité réduite du résidu de la réaction de craquage.

On observe également que la température de réaction de l'essai 3 réalisé avec un temps de séjour de 6h, en comparaison avec l'essai 2 réalisé avec un temps de séjour de 3,5h, est bien plus faible, pour obtenir un résidu de craquage similaire. A température plus élevée, la viscosité du résidu augmente fortement et rend difficile le transport du liquide. La conséquence de la diminution de température utilisable à temps de séjour plus élevé sans augmentation exagérée de viscosité est une réduction importante des performances de craquage.

### Exemple 4 : Condensation partielle d'un flux issu de réaction de craquage de lourds AA et lourds AE

Un mélange liquide, provenant de la condensation totale d'une opération de craquage d'un mélange de LAA (60%) et de LAE (40%), est vaporisé et condensé partiellement dans un condenseur à calandre et tube, à une température de 160°C. On obtient un flux liquide condensé qui représente 30% en masse du flux d'alimentation du condenseur. Ce condensat liquide a une composition représentative du reflux (5) de la Figure 1, contenant 35,4% d'AA, 0,3% d'AE, 4,4% d'AA2, 0,4% d'AA3, 11,5% d'AEP, 22% d'EPE, 6,2% d'APE, 0,1% d'HPE, 0,3% d'HQ, 0,04% de PTZ.

### Exemples 5 à 8 : Craquage avec condensation partielle et recyclage du flux condensé dans la phase liquide du réacteur

Ces exemples sont destinés à illustrer les conditions opératoires du procédé selon l'invention de la Figure 1. Le Tableau 2 résume les conditions opératoires et performances de ces différents essais.

En complément des définitions décrites plus haut, le "taux de conversion utile" est le ratio massique de la somme des composés valorisables (AA, AE et éthanol) générés par craquage et récupérés dans le flux condensé totalement (flux 4), après soustraction de la somme de ces composés présents dans l'alimentation (flux 1) et dans le flux recyclé (flux 5), rapportée au débit d'alimentation du réacteur (flux1) après soustraction des composés valorisables présents dans ce flux (1).

Le montage expérimental est le même que celui de l'exemple 1, hormis le fait que les vapeurs générées lors de la réaction de craquage sont envoyées directement dans le condenseur total (E2) et un débit de flux de condensat obtenu dans l'exemple 3 est introduit par canne plongeante dans le réacteur, en complément de l'alimentation de LAA et LAE. Ce dispositif permet de simuler de façon rigoureuse le recyclage du flux de craqueur condensé à température de 160°C, selon le ratio de débit de recyclage / débit d'alimentation de LAA et LAE de 22% attendu correspondant à un taux de condensation des vapeurs de 30%.

Dans les exemples 5, 6 et 7 (temps de séjour de 3h), le réacteur est alimenté par le mélange de LAA et LAE décrit dans l'exemple 1, à un débit de 80 g/h, et le flux de condensat de l'exemple 3 (flux 5) est introduit dans le réacteur, par canne plongeant dans la phase liquide, à un débit de 17,6 g/h. Dans l'exemple 8 (temps de séjour de 6h), le débit d'alimentation (1) de LAA et LAE est de 40 g/h et le débit de condensat (5) recyclé est de 8,8 g/h.

Après un fonctionnement en condition stable de plus de 24h, les échantillons de flux issus du condenseur total E2 (flux 4) et de résidu en fond de réacteur (flux 6) sont prélevés et analysés.

### Exemples 9 et 10 (comparatifs) : Craquage sans condensation, selon l'art antérieur

Le montage utilisé et les conditions opératoires sont les mêmes que celles décrites dans les exemples 5 à 8, hormis le fait que le recyclage de flux issu d'une condensation partielle (flux 5) a été supprimé. Comme pour les exemples 5 à 7, le débit d'alimentation en LAA et LAE de l'exemple 9 (temps de séjour de 3h) est de 80 g/h, et celui de l'exemple 10 (temps de séjour de 3h) est le même que celui de l'exemple 8, soit 40g/h.

Le Tableau 2 ci-dessous résume les conditions opératoires et performances de ces essais.

**[Tableau 2]**

| | **selon l'invention** | | | | **comparatif** | |
|---|---|---|---|---|---|---|
| N° essai | **5** | **6** | **7** | **8** | **9** | **10** |
| Temps de séjour | 3 h | 3 h | 3 h | 6 h | 3 h | 6 h |
| Température réaction | 200°C | 204°C | 207°C | 194°C | 196°C | 186°C |
| Ratio d'étêtage | 60,6% | 64,9% | 68,7% | 65,8% | 67,5% | 54,2% |
| Taux de Récupération Utile | 47,8% | 51,3% | 53,1% | 48,3% | 44,9% | 37,2% |
| Taux de Conversion Utile | 45,6% | 49,8% | 52,0% | 46,2% | 38,3% | 29,7% |
| Viscosité du résidu en Pa.s @ 100°C | 0,064 | 0,042 | 0,090 | 0,107 | 0,499 | 0,132 |

Ces exemples montrent qu'il est possible d'améliorer significativement le rendement de récupération des produits valorisables en réalisant une condensation partielle des vapeurs de craquage et en recyclant la partie condensée dans réacteur, dans un montage très simple mettant en oeuvre un condenseur opéré à la même pression que la réaction de craquage, tout en obtenant une viscosité du résidu de craquage plus faible que dans l'art antérieur.

Les essais 9 et 10 permettent de comparer les performances de craquage à différents temps de séjour. Pour une viscosité équivalente du résidu, la comparaison des essais 7 et 8 montre que la température de réaction de craquage doit être réduite lorsque le temps de séjour est plus élevé, mais les performances de récupération restent tout de même bien meilleures dans ces conditions de temps de séjour plus élevé (essai 8) que dans l'essai 9 qui a été réalisé selon l'art antérieur dans des conditions opératoires pourtant plus favorables de temps de séjour plus faible, avec une viscosité du résidu qui est en outre plus faible. Ceci montre donc que le procédé selon l'invention permet de continuer de récupérer beaucoup plus de produit noble à partir d'un même mélange de LAA-LAE, même lorsque le débit de lourds à traiter est plus faible pour des raisons de fluctuation des allures de production des ateliers d'AA et d'ester qui entrainent une augmentation du temps de séjour.

## Revendications

1. Procédé de régénération d'un mélange de sous-produits lourds provenant d'une unité de production d'acide acrylique et de sous-produits lourds provenant d'une unité de production d'ester acrylique de formule CH₂=CH-C(=O)-OR, R étant un groupement alkyle en C1-C8, ledit procédé comprenant les étapes suivantes :
i. introduire ledit mélange dans un réacteur et le soumettre à un craquage thermique produisant un flux de tête gazeux et un flux de pied (résidu) concentré en produits lourds,
ii. soumettre ledit flux de tête à une condensation partielle conduisant à l'obtention d'un flux de tête contenant de l'acide acrylique, de l'ester acrylique et de l'alcool R-OH, et d'un flux de pied,
iii. récupérer ledit résidu en vue d'un traitement d'élimination.

2. Procédé selon la revendication 1, dans lequel les étapes i) et ii) sont réalisées à la même pression absolue, comprise de préférence entre 200 et 2000 hPa.

3. Procédé selon l'une des revendications 1 et 2, dans lequel la température de craquage est comprise entre 140 et 260°C, de préférence entre 180 et 210°C.

4. Procédé selon l'une des revendications 1 à 3, dans lequel le temps de séjour du mélange réactionnel dans le réacteur de craquage est compris entre 0,5h et 10h, de préférence entre 2h et 7h.

5. Procédé selon l'une des revendications 1 à 4, dans lequel le flux de pied du réacteur (résidu) obtenu à l'issue de l'opération de craquage thermique présente une viscosité dynamique mesurée à 100°C inférieure à 1 Pa.s.

6. Procédé selon l'une quelconque des revendications 1 à 5, dans lequel le flux de pied du condenseur partiel est au moins en partie recyclé dans le réacteur.

7. Procédé selon l'une quelconque des revendications 1 à 6, dans lequel ledit flux de tête issu du condenseur partiel et contenant de l'acide acrylique, des esters acryliques et alcools R-OH est recyclé dans une unité de fabrication d'ester acrylique.

8. Procédé selon l'une quelconque des revendications 1 à 6, dans lequel ledit flux de tête issu du condenseur partiel et contenant de l'acide acrylique, des esters acryliques et alcools R-OH est recyclé dans une unité de fabrication d'ester acrylique, sous forme liquide, après condensation dans un deuxième condenseur.

9. Procédé selon l'une quelconque des revendications précédentes, dans lequel l'ester est de l'acrylate d'éthyle et l'alcool est l'éthanol.

10. Procédé selon l'une des revendications précédentes dans lequel le ratio massique flux condensé / flux gazeux entrant dans le condenseur est compris entre 5% et 50%, de préférence entre 10% et 40%.

11. Procédé selon l'une des revendications précédentes dans lequel le ratio massique Lourds AA / Lourds EA est compris entre 3/7 et 9/1, de préférence entre 1/1 et 9/1.

12. Procédé selon l'une des revendications précédentes dans lequel au moins un inhibiteur de polymérisation est introduit au niveau du condenseur partiel.

13. Procédé selon l'une des revendications 1 à 12 dans lequel la réaction de craquage thermique a lieu en l'absence de catalyseur.

14. Procédé selon l'une des revendications 1 à 12 dans lequel la réaction de craquage thermique a lieu en présence d'un catalyseur choisi parmi l'acide sulfurique, les acides sulfoniques, l'acide phosphorique, zéolites, alumines, catalyseurs à base de silice et alumine, titanates, bases, métaux alcalins, sels de métaux alcalins, amines, phosphines, ou leur combinaison.

## Patentansprüche

1. Verfahren zum Regenerieren einer Mischung von schwerflüchtigen Nebenprodukten, die aus einer Einheit zur Herstellung von Acrylsäure stammen, und von schwerflüchtigen Nebenprodukten, die aus einer Einheit zur Herstellung von Acrylsäureester mit der Formel CH₂=CH-C(=O)-OR stammen, wobei R für eine C1-C8-Alkylgruppe steht, wobei das Verfahren die folgenden Schritte umfasst :
i. Einleiten der Mischung in einen Reaktor und Einwirkenlassen eines thermischen Crackvorgangs auf dieselbe, wobei ein kopfseitiger gasförmiger Stoffstrom sowie ein sumpfseitiger Stoffstrom (Rückstand) entstehen, wobei letzterer an schwerflüchtigen Stoffen angereichert ist,
ii. Einwirkenlassen einer teilweisen Kondensation auf den kopfseitigen Stoffstrom, wobei ein kopfseitiger Stoffstrom, welcher Acrylsäure, Acrylsäureester und Alkohol R-OH enthält, und ein sumpfseitiger Stoffstrom entstehen,
iii. Auffangen des Rückstands, um diesen derart zu behandeln, dass er beseitigt wird.

2. Verfahren nach Anspruch 1, wobei die Schritte i) und ii) bei demselben Absolutdruck durchgeführt werden, welcher vorzugsweise im Bereich von 200 bis 2000 hPa liegt.

3. Verfahren nach einem der Ansprüche 1 und 2, wobei die Temperatur des Crackvorgangs im Bereich von 140 bis 260 °C, vorzugsweise von 180 bis 210 °C, liegt.

4. Verfahren nach einem der Ansprüche 1 bis 3, wobei die Verweildauer der Reaktionsmischung im Crackreaktor im Bereich von 0,5 Std. bis 10 Std., vorzugsweise von 2 Std. bis 7 Std., liegt.

5. Verfahren nach einem der Ansprüche 1 bis 4, wobei der sumpfseitige Stoffstrom des Reaktors (Rückstand), wie er nach Abschluss des thermischen Crackvorgangs erhalten wird, eine dynamische Viskosität aufweist, die im Falle einer Messung bei 100 °C weniger als 1 Pa.s beträgt.

6. Verfahren nach einem beliebigen der Ansprüche 1 à 5, wobei der sumpfseitige Stoffstrom der Vorrichtung zur teilweisen Kondensation zumindest teilweise in den Reaktor zurückgeführt wird.

7. Verfahren nach einem beliebigen der Ansprüche 1 bis 6, wobei der kopfseitige Stoffstrom, welcher aus der Vorrichtung zur teilweisen Kondensation stammt und Acrylsäure, Acrylsäureester und Alkohole R-OH enthält, in eine Einheit zur Herstellung von Acrylsäureester zurückgeführt wird.

8. Verfahren nach einem beliebigen der Ansprüche 1 bis 6, wobei der kopfseitige Stoffstrom, welcher aus der Vorrichtung zur teilweisen Kondensation stammt und Acrylsäure, Acrylsäureester und Alkohole R-OH enthält, in flüssiger Form, nachdem der in einer zweiten Kondensationsvorrichtung kondensiert wurde, in eine Einheit zur Herstellung von Acrylsäureester zurückgeführt wird.

9. Verfahren nach einem beliebigen der vorhergehenden Ansprüche, wobei es sich bei dem Ester um Ethylacrylat und bei dem Alkohol um Ethanol handelt.

10. Verfahren nach einem der vorhergehenden Ansprüche, wobei das Massenverhältnis von kondensiertem Stoffstrom / gasförmigem Stoffstrom, beim Eintritt in die Kondensationsvorrichtung im Bereich von 5 % bis 50 %, vorzugsweise von 10 % bis 40 %, liegt.

11. Verfahren nach einem der vorhergehenden Ansprüche, wobei das Massenverhältnis von schwerflüchtigen AS-Stoffen / schwerflüssigen AE-Stoffen im Bereich von 3/7 bis 9/1, vorzugsweise von 1/1 bis 9/1, liegt.

12. Verfahren gemäß einem der vorhergehenden Ansprüche, wobei im Bereich der Vorrichtung zur teilweisen Kondensation mindestens ein Polymerisationshemmer eingeleitet wird.

13. Verfahren nach einem der Ansprüche 1 bis 12, wobei die thermische Crackreaktion ohne Vorliegen von Katalysatoren erfolgt.

14. Verfahren nach einem der Ansprüche 1 bis 12, wobei die thermische Crackreaktion in Gegenwart eines Katalysators erfolgt, der aus Schwefelsäure, Sulfonsäuren, Phosphorsäure, Zeolithen, Aluminiumoxiden, Katalysatoren auf Basis von Siliciumdioxid und Aluminiumoxid, Titanaten, Basen, Alkalimetallen, Alkalimetallsalzen, Aminen, Phosphinen oder deren Kombinationen ausgewählt ist.

## Claims

1. A process for the regeneration of a mixture of heavy byproducts originating from a unit for the production of acrylic acid and of heavy byproducts originating from a unit for the production of acrylic ester of formula CH₂=CH-C(=O)-OR, R being a C₁-C₈ alkyl group, said process comprising the following stages:
i. introducing said mixture into a reactor and subjecting it to a thermal cracking, producing a gaseous top stream and a bottom stream (residue) concentrated in heavy products,
ii. subjecting said top stream to a partial condensation, resulting in a top stream being obtained containing acrylic acid, acrylic ester and alcohol R-OH and in a bottom stream being obtained,
iii. recovering said residue for the purpose of a removal treatment.

2. The process as claimed in claim 1, in which stages i) and ii) are carried out at the same absolute pressure, preferably of between 200 and 2000 hPa.

3. The process as claimed in either of claims 1 and 2, in which the cracking temperature is of between 140°C and 260°C, preferably between 180°C and 210°C.

4. The process as claimed in one of claims 1 to 3, in which the residence time of the reaction mixture in the cracking reactor is of between 0.5 h and 10 h, preferably between 2 h and 7 h.

5. The process as claimed in one of claims 1 to 4, in which the bottom stream from the reactor (residue) obtained on conclusion of the thermal cracking operation exhibits a dynamic viscosity, measured at 100°C, of less than 1 Pa.s.

6. The process as claimed in any one of claims 1 to 5, in which the bottom stream from the partial condenser is at least partially recycled to the reactor.

7. The process as claimed in any one of claims 1 to 6, in which said top stream resulting from the partial condenser and containing acrylic acid, acrylic esters and alcohols R-OH is recycled to a unit for the manufacture of acrylic ester.

8. The process as claimed in any one of claims 1 to 6, in which said top stream resulting from the partial condenser and containing acrylic acid, acrylic esters and alcohols R-OH is recycled to a unit for the manufacture of acrylic ester, in the liquid form, after condensation in a second condenser.

9. The process as claimed in any one of the preceding claims, in which the ester is ethyl acrylate and the alcohol is ethanol.

10. The process as claimed in one of the preceding claims, in which the ratio by weight of condensed stream to gas stream entering the condenser is of between 5% and 50%, preferably between 10% and 40%.

11. The process as claimed in one of the preceding claims, in which the AA heavy products/AE heavy products ratio by weight is of between 3/7 and 9/1, preferably between 1/1 and 9/1.

12. The process as claimed in one of the preceding claims, in which at least one polymerization inhibitor is introduced at the partial condenser.

13. The process as claimed in one of claims 1 to 12, in which the thermal cracking reaction takes place in the absence of catalyst.

14. The process as claimed in one of claims 1 to 12, in which the thermal cracking reaction takes place in the presence of a catalyst chosen from sulfuric acid, sulfonic acids, phosphoric acid, zeolites, aluminas, catalysts based on silica and alumina, titanates, bases, alkali metals, alkali metal salts, amines, phosphines, or their combination.
